(19) Europäisches Patentamt
European Patent Office
Office européen des brevets

(11) Veröffentlichungsnummer: **0 025 069**
**B1**

(12) # EUROPÄISCHE PATENTSCHRIFT

(45) Veröffentlichungstag der Patentschrift: **16.06.82**

(21) Anmeldenummer: **79103315.2**

(22) Anmeldetag: **06.09.79**

(51) Int. Cl.³: **A 61 K 31/435,**
C 07 D 471/22
//(C07D471/22, 221/00,
221/00, 221/00, 221/00)

(54) **Neues Pentylspartein-Salz, dieses enthaltendes Arzneimittel und Verfahren zur Herstellung eines solchen Arzneimittels.**

(43) Veröffentlichungstag der Anmeldung:
**18.03.81 Patentblatt 81/11**

(45) Bekanntmachung des Hinweises auf die Patenterteilung:
**16.06.82 Patentblatt 82/24**

(84) Benannte Vertragsstaaten:
**AT BE CH DE FR GB IT LU NL SE**

(56) Entgegenhaltungen:
**DE - A - 2 360 475**
**CHEMICAL ABSTRACTS, Band 50, 1956, Zusammenfassung 1048e, Columbus, Ohio, US, T. KYOSUKE et al.: "Synthesis of lupine alkaloids. I. Synthesis of a new isomer of sparteine".**

(73) Patentinhaber: **Kali-Chemie Pharma GmbH**
**Hans-Böckler-Allee 20 Postfach 220**
**D-3000 Hannover 1 (DE)**

(72) Erfinder: **Hachmeister, Bernd, Dr.rer.nat., Dipl.-Chem.**
**Laurinweg 18A**
**D-3000 Hannover (DE)**
Erfinder: **Milkowski, Wolfgang, Dr.rer.nat., Dipl.-Chem.**
**Fasanenweg 13**
**D-3167 Burgdorf (DE)**
Erfinder: **Kühl, Ulrich, Dr.med.vet.**
**Bergener Strasse 9**
**D-3000 Hannover (DE)**
Erfinder: **Buschmann, Gerd, Dr.med.vet.**
**Matthäikirchstrasse 27**
**D-3000 Hannover (DE)**
Erfinder: **Budden, Renke, Dr.med.vet.**
**Rodewaldstrasse 18**
**D-3000 Hannover (DE)**

(74) Vertreter: **Lauer, Dieter, Dr.**
**c/o Kali-Chemie Aktiengesellschaft Postfach 220**
**D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 025 069 B1

# Neues Pentylspartein-Salz, dieses enthaltendes Arzneimittel und Verfahren zur Herstellung eines solchen Arzneimittels

Die Erfindung betrifft ein neues Pentylspartein-Salz, ein dieses enthaltendes Arzneimittel und ein Verfahren zur Herstellung eines solchen Arzneimittels.

Das aus dem Besenginster gewinnbare Alkaloid Spartein ist bereits seit langem für seine guten pharmakologischen eigenschaften bekannt. So wird es z.B. als Venentherapeutikum und als herzwirksames Mittel eingesetzt.

Die in 17-Stellung alkylsubstituierten Derivate des Sparteins mit der allgemeinen Formel

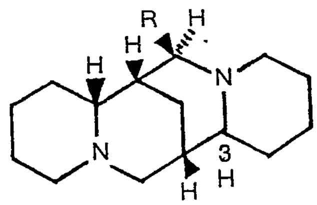

In der R eine Alkylgruppe mit 1—10 C-Atomen bedeutet, weisen eine gegenüber dem Spartein verbesserte pharmakologische Wirksamkeit auf (DE—OS 23 60 475) und sollen durch Umsetzung der Basen mit üblichen Säuren, wie Salzsäure, Schwefelsäure, Phosphorsäure, Gluconsäure und Perchlorsäure gut kristallisierende Salze ergeben. In den Ausführungsbeispielen der DE—OS 23 60 475 werden nur Perchlorate isoliert.

Das Perchlorat des 17-$\beta$-n-Pentysparteins—ein stabiles Salz—scheidet aus mehreren Gründen aus, bei der Herstellung von Arzneimitteln verwendet zu werden. Zum einen bestehen bei der Herstellung erhebliche Sicherheitsrisiken, denn bekanntlich neigen perchlorsäurehaltige alkoholische Lösungen zu Explosionen. Aus alkoholischer Lösung wird aber das Perchlorat-Salz ausgefällt. Außerdem ist das Perchlorat in Wasser nur zu 2% (bei 22°C) löslich; damit scheidet die Applikation als wässrige Lösung aus, sei es zur peroralen oder intravenösen Anwendung.

Zum anderen bestehen gegen das Perchlorat große Bedenken hinsichtlich seiner Toxizität. So ist vom Perchlorat-Anion bekannt, daß es nach Applikation bei Menschen zu Leukopenie, Agranulozytose und aplastischer Anämie führen kann.

Versuche, stabile, kristalline Salze mit den anderen, in der DE—OS 23 60 475 angeführten Säuren zu erhalten, scheiterten, da nur das Sulfat und das Hydrochlorid des 17-$\beta$-n-Pentylsparteins als kristalline Salze anfallen, diese sich aber als extrem hygroskopisch erwiesen und damit für den Einsatz in Arzneimittelzubereitungen wenig geeignet sind.

Auch darüber hinausgehende Versuche, stabile Salze durch Kristallisation von 17-$\beta$-n-Pentylspartein mit anderen Säuren wie Benzoesäure, Essigsäure, Citronensäure, Maleinsäure, (+)Campher-10-sulfonsäure oder 3-Hydroxynaphthalincarbonsäure-2 zu erhalten führten zu keinem Erfolg.

Aufgabe der Erfindung war also, ein pharmakologisch verträgliches, stabiles Salz des 17-$\beta$-n-Pentylsparteins zur Verfügung zu stellen.

Überraschenderweise zeigte es sich, daß die Übersauren Säureadditions-Salze des 17-$\beta$-n-Pentylsparteins mit Weinsäure oder Fumarsäure diese Kriterien erfüllen. Derartige Salze des Sparteins, insbesondere des 17-$\beta$-n-Pentylsparteins waren bisher nicht bekannt.

Unter übersauren Säureadditions-Salzen werden Salze verstanden, die pro Basenmolekül mehr als 2,1 vorsugsweise mehr als 2,3 Moleküle Säure enthalten. Zur Darstellung dieser übersauren Salze ist es lediglich notwendig, eine Lösung herzustellen, die nebeneinander 17-$\beta$-n-Pentylspartein und die salzbildende Säure im Molverhältnis 1:1 bis 1:10 enthält; aus diesen Lösungen fällt das übersaure Salz kristallin aus und kann ggf. getrocknet und ggf. nach an sich bekannten Methoden gereinigt werden.

So konnten mit Fumarsäure stabile Salze erhalten werden, in denen das Molverhältnis Base:Säure im Bereich von 1:2,5 bis 1:3,0 variierte.

Besonders bevorzugte salzbildende Säure ist die L(+)-Weinsäure. Sie bildet selbst bei Variation des Base:Säure-Molverhälnisses in der Kristallisationslösung von 1:1 bis 1:10 ein übersaures Säureadditions-Salz, dessen molare Zusammensetzung lediglich im Bereich von 1:2,5 bis 1:2,8 schwankt und selbst nach mehrmaligem Umkristallisieren nicht unter das Verhältnis 1:2,3 absinkt.

Die erfindungsgemäßen übersauren Säureadditions-Salze besitzen wertvolle pharmakologische Eigenschaften. Wie die in Tabelle 1 zusammengestellten Ergebnisse zeigen, ist das übersaure 17-$\beta$-n-Pentylspartein-L(+)tartrat in seinen Eigenschaften dem Sparteinsulfat überlegen und vergleichbar dem—aus pharmakologischen Gründen nicht verwendbaren—Pentylsparteindiperchlorat.

In Tabelle 1 sind in der Spalte "fRZ" bzw. in der Spalte "Kraft" diejenigen Konzentrationen ($\mu$Mol/1) angegeben, bei denen es im Mittel zu einer Verlängerung der funktionellen Refraktärzeit (fRZ) auf 125% der Ausgangswerte, bzw. zu einer Verminderung der Kontraktionskraft (Kraft) auf 75% der Ausgangswerte kommt. Diese Werte wurden durch Messung am isolierten Meerschweinchenvorhof nach der Methode von Govier (modifiziert) ermittelt, dargestellt in W. C. Govier, J. pharmacol. exp. Ther *148*, 1000 (1965).

Die $LD_{50}$, i.p.-Werte (θMol/1) wurden durch Toxizitätsmessung an der Maus, 8 Tage nach einmaliger Applikation ermittelt. Die Berechnung der $LD_{50}$-Werte erfolgte nach J. P. Litchfield et al, J. pharmacol. exp.Ther.*96*,99(1949).

Tabelle 1:

| | übersaures Pentylspartein L(+)-tartrat | Pentylspartein-diperchlorat | Sparteinsulfat |
|---|---|---|---|
| fRZ | 9,1 | 7,7 | 33,9 |
| Kraft | 22,1 | 19,2 | 959 |
| $LD_{50}$i.p. | 262 | 283 | 202 |

Weitere, besondere Vorteile der übersauren Säureadditions-Salze liegen außer in ihrer guten Stabilität in ihrer hervorragenden Wasserlöslichkeit. So ist das 17-β-n-Pentyl-L(+)-tartrat bei 22°C zu mehr als 100 g in 100 g Wasser löslich. Auch die Lösungen der übersauren Säureadditions-Salze in Wasser zeichnen sich durch gute Stabilität aus.

Zur Herstellung von übersaure Säureadditions-Salze enthaltenden Arzneimitteln werden die Salze zuzammen mit üblichen Träger- und/ oder Hilfsstoffen in an sich bekannter Weise in geeignete galenische Applikationsformen überführt.

Beispiele 1 bis 11

Die Beispiele 1 bis 10 zeigen die Herstellung von übersauren Säureadditionssalzen, Beispiel 11 zeigt ein—nicht erfindungsgemäßes—Vergleichsbeispiel.

Gemäß den Angaben in Tabelle 2 wird 17-β-n-Pentylspartein in X ml heißem Lösungsmittel gelöst. Hierzu gibt man eine heiße Lösung der Säure in Y ml heißem Lösungsmittel. Der nach dem Abkühlen kristallisierte weiße Niederschlag wird abgesaugt, mit wenig kaltem Lösungsmittel gewaschen und bei 120°C im Vakuum getrocknet.

Zur Bestimmung der Salzzusammensetzung wurde der Gehalt an 17-β-n-Pentylspartein-Base titrimetrisch mit Perchlorsäure in Eisessig/Acetanhydrid bestimmt. Die Säure wurde in Wasser/Isopropanol mit Natronlauge titriert.

Tabelle 2:
Herstellung von übersauren Säureadditions-Salzen

| Bsp. | Säuretyp | Menge Base [g] | Menge Säure [g] | Molverh. Vorgabe [Base:Säure] | Lösungsmittel | X [ml] | Y [ml] | Ausbeute Salz [g] | Ausbeute Salz [%] | Molverh. Salz [Base:Säure] | Fp [°C] |
|---|---|---|---|---|---|---|---|---|---|---|---|
| 1 | L(+)-Weinsäure | 9,13 | 11,5 | 1 : 2,55 | Äthanol | 30 | 50 | 19,9 | 98 | 1 : 2,55 | 176—178 |
| 2 | L(+)-Weinsäure | 9,13 | 13,5 | 1 : 3 | Äthanol | 100 | 100 | 19,3 | 90 | 1 : 2,74 | |
| 3 | L(+)-Weinsäure | | Nr. 2 3 × aus Äthanol umkristallisiert | | | | | | | 1 : 2,37 | |
| 4 | L(+)-Weinsäure | 1,7 | 4,2 | 1 : 5 | Äthanol | 20 | 20 | 3,8 | 94,6 | 1 : 2,76 | 177 |
| 5 | L(+)-Weinsäure | 1,7 | 8,4 | 1 : 10 | Äthanol | 20 | 40 | 3,0 | 78,9 | 1 : 2,51 | 180 |
| 6 | L(+)-Weinsäure | 1,52 | 1,5 | 1 : 2 | Isopropanol | 20 | 20 | 2,2 | 79 | 1 : 2,52 | |
| 7 | L(+)-Weinsäure | 1,52 | 1,5 | 1 : 2 | Aceton | 20 | 20 | 2,1 | 76 | 1 : 2,5 | |
| 8 | D(—)-Weinsäure | 3,05 | 3 | 1 : 2 | Äthanol | 30 | 30 | 5,05 | 75 | 1 : 2,46 | |
| 9 | Fumarsäure | 3,05 | 2,32 | 1 : 2 | Äthanol | 30 | 30 | 4,38 | 73 | 1 : 2,55 | 145 |
| 10 | Fumarsäure | 3,05 | 2,32 | 1 : 2 | Äthanol | 30 | 30 | 4,03 | 62 | 1 : 2,98 | |
| 11 | Schwefelsäure | 3,05 | 1,96 | 1 : 2 | Äthanol | 30 | 8 | Es wurden schmierige Kristalle erhalten, die sich beim Absaugen durch Aufnahme von Luftfeuchtigkeit sehr schnell auflösten. | | | |

Zur Herstellung des 17-$\beta$-n-Pentylsparteins wurde gemäß DE—OS 23 60 475 gearbeitet; das dazu notwendige 17-Hydroxyspartein wurde gemäß dem Verfahren der deutschen Patentan meldung P 28 25 117 hergestellt.

Die in Beispiel 1 bis 10 hergestellten übersauren Säure-Additionssalze und auch ihre 1%igen wässrigen Lösungen weisen gute Stabilität auf. So zeigt sich bei dünnschichtchromatographischer Untersuchung keinerlei Zersetzung, weder nach einmonatiger Lagerung bei 55°C, noch bei Belichtung (30 Stunden, Schnellbelichtungsgerät Suntest, Firma Heraeus).

Die Beispiele 12 bis 14 zeigen galenische Zubereitungen unter Verwendung des 17-$\beta$-n-Pentylsparteins aus Versuch 1. Unter "Teile" sind in allen Beispielen "Gewichtsteile" zu verstehen.

Beispiel 12

TABLETTEN

| | |
|---|---|
| 17-$\beta$-n-Pentylspartein-L(+)-tartrat | 75 Teile |
| Lactose | 250 Teile |
| Maisstärke | 160 Teile |
| Lösliche Stärke | 10 Teile |
| Magnesiumstearat | 5 Teile |
| Total | 500 Teile |

*Herstellungsvorschrift:*

17-$\beta$-n-Pentylspartein-L(+)-tartrat, Lactose und Maisstärke werden im LÖDIGE-Mischer gründlich vermischt und dann mit einer 20%igen wäßrigen Lösung der löslichen Stärke granuliert. Die feuchte Mass wird durch eine 1,6 mm-Sieb passiert und anschließend im Wirbelschichttrockner bei einer Zuluft von 70°C auf einen Wassergehalt von ca. 4% getrocknet. Das so erhaltene Granulat wird durch ein 1 mm-Sieb passiert, mit Magnesiumstearat vermischt und zu Tabletten vom Durchmesser 12 mm verpreßt. Das Gewicht jeder Tablette beträgt im Mittel 500 mg, so daß jeweils 75 mg Wirkstoff in der Einzeldosis enthalten sind.

Beispiel 13

TABLETTEN RETARDFORM

| | |
|---|---|
| 17-$\beta$-n-Pentylspartein-L(+)-tartrat | 300 Teile |
| Aerosil | 5 Teile |
| Hydriertes Rizinusöl | 100 Teile |
| Methylhydroxypropylcellulose | 50 Teile |
| Talkum | 45 Teile |
| Total | 500 Teile |

*Herstellungsvorschrift:*

17-$\beta$-n-Pentylspartein-L(+)-tartrat, hydriertes Rizinusöl, Aerosil und Talkum werden im LÖDIGE-Mischer gründlich gemischt und mit einer Lösung von Methylhydroxypropylcellulose in einem Gemisch von Methylenchlorid/Äthanol (60:40) granuliert. Die feuchte Masse wird durch ein 1,6 mm-Sieb passiert und dann auf Horden über Nacht bei 40°C getrocknet. Das getrocknete Produkt wird nochmals durch ein 1,6 mm-Sieb passiert und zu Kernen von 11 m Durchmesser verpreßt. Das Gewicht jedes Kernes beträgt im Mittel 500 mg, so daß jeweils 300 mg Wirkstoff in der Einzeldosis enthalten sind.

Die Kerne werden schließlich nach bekannten Verfahren mit einer Hülle von Eudragit RS (10 mg pro Kern) versehen. (Eudragit RS ist ein Copolymerisat der Firma RÖHM, bestehend aus Trimethylammoniumäthylmethacrylatchlorid, Methacrylsäuremethylester und Acrylsäureäthylester im Verhältnis 5:65:30).

Beispiel 14

AMPULLEN

| | |
|---|---|
| 17-β-n-Pentylspartein-L(+)-tartrat | 75 Teile |
| Natriumchlorid | 9 Teile |
| Wasser | 916 Teile |
| Total | 1.000 Teile |

*Herstellungsvorschriften:*

17-β-n-Pentylspartein-L(+)-tartrat und Natriumchlorid werden in dem Wasser gelöst, die Lösung filtriert und nach dem Abfüllen in 1 ml Ampullenflaschen sterilisiert. Jede Ampulle enthält 75 mg der Wirksubstanz.

**Patentansprüche**

1. Übersaures Säureadditionssalz aus 17-β-n-Pentylspartein und einer Dicarbonsäure aus der Gruppe Weinsäure oder Fumarsäure, enthaltend 2,1—3,5 Mol Säure pro Mol Base.

2. Übersaures Säureadditionssalz gemäß Anspruch 1, dadurch gekennzeichnet, daß es 2,3—3,0 Mol Säure pro Mol Base enthält.

3. Übersaures Säureadditionssalz gemäß Anspruch 1 oder 2, dadurch gekennzeichnet, daß es als Dicarbonsäure L(+)-Weinsäure enthält.

4. Arzneimittelzubereitung enthaltend ein pharmakologisch verträgliches, stabiles, übersaures Säureadditonssalz aus 17-β-n-Pentylspartein und einer Dicarbonsäure aus der Gruppe Weinsäure oder Fumarsäure.

5. Arzneimittelzubereitung nach Anspruch 4, dadurch gekennzeichnet, daß im Salz Basen- und Säurerest das Molverhältnis 1 :> 2,1 bilden.

6. Arzneimittelzubereitung nach Anspruch 4, dadurch gekennzeichnet, daß im Salz Basen- und Säurerest das Molverhältnis 1 :> 2,3 bilden.

7. Arzneimittelzubereitung nach einem der Ansprüche 4, 5 oder 6, dadurch gekennzeichnet, daß die salzbildende Säure L(+)-Weinsäure ist.

8. Verfahren zur Herstellung eines übersauren Säureadditionssalzes gemäß Anspruch 1, dadurch gekennzeichnet, daß man

a) das Salz aus einer Lösung kristallisieren läßt, die 17-β-n-Pentylspartein und die salzbildende Säure im Molverhältnis 1:1 bis 1:10 enthält,

b) das Salz gegebenenfalls trocknet und gegebenenfalls nach an sich bekannten Methoden reinigt.

9. Verfahren nach Anspruch 8, dadurch gekennzeichnet, daß als salzbildende Säure L(+)-Weinsäure eingesetzt wird.

10. Verfahren zur Herstellung einer Arzeimittel-zubereitung gemäß einem der Ansprüche 4—7, dadurch gekennzeichnet, daß man das übersaure Säureadditionssalz zusammen mit üblichen Träger- und/oder Hilfsstoffen in eine galenische Applikationsform überführt.

**Revendications**

1. Sel suracide d'addition d'acide de 17-β-n-pentylspartéine et d'un acide dicarboxylique choisi parmi le groupe comprenant l'acide tartrique et l'acide fumarique, ce sel contenant 2,1 à 3,5 moles d'acide par mole de base.

2. Sel suracide d'addition d'acide suivant la revendication 1, caractérisé en ce qu'il contient 2,3 à 3 moles d'acide par mole de base.

3. Sel suracide d'addition d'acide suivant la revendication 1 ou 2, caractérisé en ce que, comme acide dicarboxylique, il contient l'acide L(+)-tartrique.

4. Préparation médicamenteuse contenant un sel suracide d'addition d'acide stable, pharmacologiquement compatible et constitué de 17-β-n-pentylspartéine et d'un acide dicarboxylique choisi parmi le groupe comprenant l'acide tartrique et l'acide fumarique.

5. Préparation médicamenteuse suivant la revendication 4, caractérisé en ce que, dans le sel, le radical base et le radical acide forment un rapport molaire de 1:>2,1.

6. Préparation médicamenteuse suivant la revendication 4, caractérisée en ce que, dans le sel, le radical base et le radical acide forment le rapport molaire 1:>2,3.

7. Préparation médicamenteuse suivant une des revendications 4, 5 et 6, caractérisée en ce que l'acide formateur de sel est l'acide L(+)-tartrique.

8. Procédé de préparation d'un sel suracide d'addition d'acide suivant la revendication 1, caractérisé en ce que:

a) on laisse cristalliser le sel dans une solution contenant la 17-β-n-pentylspartéine et l'acide formateur de sel dans le rapport molaire de 1:1 à 1:10,

b) on sèche éventuellement le sel et on le purifie éventuellement suivant des méthodes connues en soi.

9. Procédé suivant la revendication 8, caractérisé en ce que, comme acide formateur de sel, on utilise l'acide L(+)-tartrique.

10. Procédé en vue d'obtenir une préparation médicamenteuse suivant une des revendications 4 à 7, caractérisé en ce qu'on transforme le sel suracide d'addition d'acide sous une forme d'application galénique avec des substances supports et/ou des substances auxiliaires habituelles.

**Claims**

1. Superacid acid addition salt of 17-β-n-pentylsparteine and a dicarboxylic acid of the group tartaric acid or fumaric acid, containing 2.1—3.5 mol acid per mol base.

2. Superacid acid addition salt according to Claim 1, characterised in that it contains 2.3—3.0 mol acid per mol base.

3. Superacid acid addition salt according to Claim 1 or 2, characterised in that it contains L (+)—tartaric acid as the dicarboxylic acid.

4. Pharmaceutical preparation containing a pharmacologically well-tolerated, stable, superacid acid addition salt of 17-β-n-pentylsparteine and a dicarboxylic acid of the group tartaric acid or fumaric acid.

5. Pharmaceutical preparation according to Claim 4, characterized in that in the salt the base and acid radicals are present in the molar ratio 1:>2.1.

6. Pharmaceutical preparation according to Claim 4, characterized in that in the salt the base and acid radicals are present in the molar ratio 1:>2.3.

7. Pharmaceutical preparation according to one of Claims 4, 5 or 6, characterized in that the salt-forming acid is L (+)—tartaric acid.

8. Process for the production of a superacid acid addition salt according to Claim 1, characterised in that

a) the salt is allowed to crystallize from a solution, which contains 17-β-n-pentylsparteine and the salt-forming acid in the molar ratio 1:1 to 1:10,

b) the salt is dried, if necessary and, if necessary, is purified according to methods known per se.

9. Process according to Claim 8, characterized in that L (+)—tartaric acid is used as salt-forming acid.

10. Process for the production of a pharmaceutical preparation according to one of Claims 4 to 7, characterized in that the superacid acid addition salt together with conventional carrier and/or auxiliary substances is converted into a form suitable for pharmaceutical administration.